(19) Europäisches Patentamt
European Patent Office
Office européen des brevets

(11) **EP 4 484 452 A1**

(12) **EUROPEAN PATENT APPLICATION**
published in accordance with Art. 153(4) EPC

(43) Date of publication:
**01.01.2025 Bulletin 2025/01**

(21) Application number: **23760335.2**

(22) Date of filing: **17.02.2023**

(51) International Patent Classification (IPC):
*C08B 37/08* (2006.01)        *C08J 3/28* (2006.01)
*A61K 8/73* (2006.01)        *A61Q 19/00* (2006.01)

(86) International application number:
**PCT/KR2023/002352**

(87) International publication number:
**WO 2023/163461 (31.08.2023 Gazette 2023/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.02.2022 KR 20220023658**

(71) Applicant: **Scai Therapeutics Co., Ltd.
Seoul 06524 (KR)**

(72) Inventors:
• **KIM, Chul Hwan
Daejeon 35246 (KR)**
• **KIM, Kyoung Hee
Daejeon 35246 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **STRUCTURE OF POLYMERIC HYALURONIC ACID, METHOD FOR PREPARING SAME, AND COSMETIC COMPOSITION COMPRISING SAME**

(57)    The present invention relates to a structure of polymeric hyaluronic acid formed by applying shear stress to a solution containing polymeric hyaluronic acid, a preparation method thereof, and a cosmetic composition containing the same.

【Figure 3】

50 nm

EP 4 484 452 A1

**Description**

[Technical Field]

The present application claims the benefit of priority based on Korean Patent Application No. 10-2022-0023658 filed on February 23, 2022, the entire contents of which are incorporated herein as part of this specification.
The present invention relates to the structure of polymeric hyaluronic acid, a preparation method thereof, and a cosmetic composition containing the same, and more specifically, to the structure of polymeric hyaluronic acid whose viscosity has been reduced by applying shear stress to the polymeric hyaluronic acid, a preparation method thereof, and a cosmetic composition containing the same.

[Background Art]

High molecules are used in many industrial fields, and are applied in a very wide range of fields. Types of high molecules can be broadly divided into synthetic high molecules and natural high molecules. Among these, natural high molecules are high molecules based on sugars such as hyaluronic acid and cellulose, and they are materials with numerous applications, for example, which are mainly used for drug release or as gel components in various cosmetic compositions.
Hyaluronic acid is a biopolymer present in the human body, which is widely distributed in skin, vitreous body of the eye, joint fluid, muscle, umbilical cord, cockscomb, etc., and exerts various functions in each part of the body. For example, hyaluronic acid prevents the invasion of bacteria into the human body or acts as a lubricant to facilitate the movement of the body. Hyaluronic acid is isolated and extracted from animal tissues or raw materials, and has a structure in the form of sodium salt. Hyaluronic acid improves the density of the skin by promoting the synthesis of collagen, or since it has a moisture absorption capacity of up to 1,000 times its weight and thus has a strong moisturizing effect, it is widely developed as cosmetics, quasi-drugs, and bio materials.
It is known that in the raw materials of cosmetics where solutions of biopolymer are used, there are several physical properties that define the flow of the solution, and the flow characteristics of polymer solutions such as viscosity, elasticity (G'), viscous property (G"), complex viscosity (G*), and phase angle (P/A) affect the feeling of use and skin penetration. For example, if the viscosity of the polymer solution is too low, there is no feeling of elasticity, thereby giving a feeling of use of the property of flowing like water, and if the viscosity of the polymer solution is too high, it is very sticky and has too much elasticity and thus it has poor spreadability or does not apply well when applied to the skin.
In the case of existing cosmetic formulations containing hyaluronic acid, most of them were used at a low concentration of 0.01% to 0.02% due to the high viscosity and high price of hyaluronic acid. In the case of products containing hyaluronic acid in high concentrations of 0.05% or more, they could only be used in the form of essence or emulsion. In general, most functional cosmetic raw materials can be expected to exert their function only when they are contained in an amount of 2% or more in the formulation. However, the amount of hyaluronic acid contained in most existing cosmetic formulations was a very small amount of about 0.01 to 0.02%, and thus the effect of hyaluronic acid itself could not be obtained. In order to maximize the moisturizing effect due to hyaluronic acid, if it contains more than 1%, there is a problem that due to the increase in viscosity, the feeling of use is significantly decreased due to phenomena such as skin pulling during use, or it remains in the form of a film on the surface of the skin over time after application.

[Prior art Document]

[Patent Document]

(Patent Document 1) Korean Laid-open Patent Publication No. 10-2021-0015377 (2021.02.10), A METHOD FOR PREPARING HYALURONIC ACID WITH LOW VISCOSITY

[Disclosure]

[Technical Problem]

The technical problem to be achieved by the present invention is to provide a structure of polymeric hyaluronic acid, a preparation method thereof, and a cosmetic composition containing the same, and more specifically to provide a structure of polymeric hyaluronic acid with reduced viscosity without reducing the molecular weight by applying shear stress to the polymeric hyaluronic acid, a preparation method thereof, and a cosmetic composition containing the same.
The technical problem to be achieved by the present invention is not limited to the mentioned technical problem, and other technical problems not mentioned will be clearly understood by those skilled in the art from the description below.

[Technical Solution]

In order to achieve the above technical problems, the structure of polymeric hyaluronic acid according to one aspect of the present invention may have a single polymer chain.

In an embodiment of the present invention, the single polymer chain may form an ionic aggregate.

In an embodiment of the present invention, the ionic aggregate may have a spherical structure with a diameter of 1 to 20 nm.

In an embodiment of the present invention, the molecular weight of the polymeric hyaluronic acid may be in the range of $5.0 \times 10^4$ to $5.0 \times 10^6$ g/mol.

In an embodiment of the present invention, the structure may be prepared by applying shear stress to a solution containing hyaluronic acid which is a precursor of the structure.

In an embodiment of the present invention, the shear stress may be applied by passing a solution containing hyaluronic acid, which is a precursor of the structure, through a column filled with silica.

In an embodiment of the present invention, the shear stress may be applied by passing a solution containing hyaluronic acid, which is a precursor of the structure, through one or more filter papers.

In an embodiment of the present invention, the filter papers may be arranged by stacking two or more filter papers.

In an embodiment of the present invention, the shear stress may be applied by applying ultrasonic waves to a solution containing hyaluronic acid which is a precursor of the structure.

In an embodiment of the present invention, when the solution containing the structure has a molecular weight of $5.0 \times 10^4$ to $5.0 \times 10^6$ g/mol and a concentration of 0.1 to 2.0%, the viscosity of the solution containing the structure may be 10 to 700 cP.

In an embodiment of the present invention, when the molecular weight and concentration of the structure and hyaluronic acid, which is a precursor of the structure, are the same, a/b, which is the ratio of the viscosity (a) of the solution containing the structure and the viscosity (b) of the solution containing hyaluronic acid, which is a precursor of the structure, may be 0.10 to 0.99.

In an embodiment of the present invention, the contact angle of the solution containing the structure of the polymeric hyaluronic acid may be 20° to 30° on the hydrophilic glass surface.

In an embodiment of the present invention, the contact angle of the solution containing the structure of the polymeric hyaluronic acid may be 80° to 100° on the hydrophobic glass surface.

In an embodiment of the present invention, the intrinsic viscosity of the solution containing the structure of the polymeric hyaluronic acid may be 0.1 to 2.0 $m^3$/kg.

In an embodiment of the present invention, it is possible to provide a cosmetic composition containing the structure of the polymeric hyaluronic acid.

In an embodiment of the present invention, it is possible to provide a preparation method of the structure of polymeric hyaluronic acid, in which the structure of polymeric hyaluronic acid is prepared by applying shear stress to a solution containing hyaluronic acid which is a precursor of the structure.

[Advantageous Effects]

By preparing a structure of hyaluronic acid with a new structure by utilizing intramolecular interactions such as ionic bonds such as hydrogen bonds and/or interactions such as ionic clusters while maintaining the same high molecular weight, the present invention has advantages that the viscosity is significantly lowered, and the exposed surface of the structure is hydrophobic and thus reduces stickiness, thereby improving the feeling of use when applied to the skin, and further improving the moisturizing properties of polymeric hyaluronic acid, and skin permeability is also improved.

Due to these advantages, the structure of polymeric hyaluronic acid of the present invention can be used in various cosmetic compositions.

[Description of Drawings]

FIG. 1 is a diagram showing the polymer chain of hyaluronic acid which is a precursor of the structure according to an embodiment of the present invention.

FIG. 2 is a diagram showing a single polymer chain of hyaluronic acid which is a precursor of the structure according to an embodiment of the present invention.

FIG. 3 is a Scanning Electron Microscope (SEM) image of an aqueous solution containing the structure according to an embodiment of the present invention.

FIG. 4 is a Scanning Electron Microscope (SEM) image of an aqueous solution containing the structure according to an embodiment of the present invention.

FIG. 5 is a Transmission Electron Microscope (TEM) image of hyaluronic acid, which is a precursor of the structure according to an embodiment of the present invention.

FIG. 6 shows a comparison of HPLC measurement results according to an embodiment of the present invention.

[Best Mode]

Generally, unlike the structure of molecules with low molecular weight, high molecules have a long chain structure with regular repeating units through chemical reactions of monomer molecules. The structure of a high molecule with a long chain exists in a solid state or, when it exists in a liquid state, it has a structure in which the chain is twisted.

In very dilute solutions of high molecules, viscosity is related to volume. This is well known as Einstein's formula, and can be expressed as Equation 1 below:

[Equation 1]

$$\eta_{rel} = \frac{\eta}{\eta_0} = 1 + 2.5\phi + f(\phi)$$

wherein $\eta$ refers to the viscosity of the solution, $\eta_0$ refers to the viscosity of the solvent, $\eta_{rel}$ refers to the relative viscosity, and $\varphi$ represents the volume ratio.

In the case of a volume of solution of high molecules, the greater the molecular weight, the greater the affinity between the solvent and the high molecule, and the greater the repulsive force between unit chain structures, the greater the volume per unit mass.

Also, in the solution of high molecules, as the concentration is increased, the chains penetrate into each other to form a very large continuum structure, and as the concentration of high molecules is diluted, the interactions between each chain is decreased.

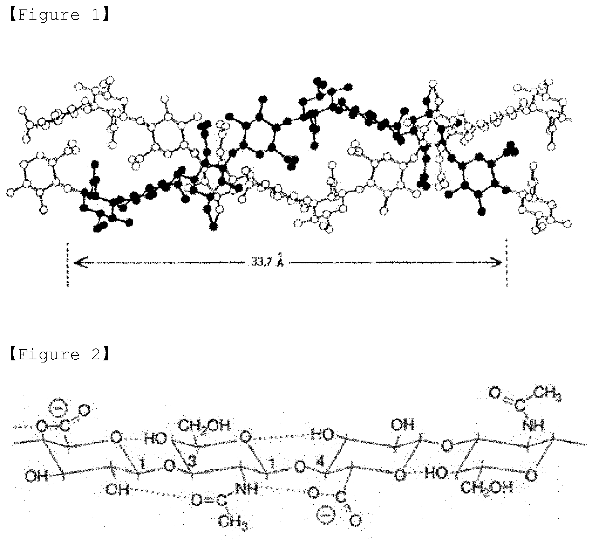

Unlike ionic polymers, where each chain exists independently, it is known that high molecules of hyaluronic acid in aqueous solution forms a twin helix structure with two chains bundled together. This is also called twin helix or macromolecular crowding. FIG. 1 shows a schematic diagram in which two chains of the high molecule of hyaluronic acid are intertwined to form a twin helix structure.

Generally, compared to low molecules, the high molecule of hyaluronic acid promotes the synthesis of collagen and has an excellent moisturizing effect due to its long residence time in the skin. However, because hyaluronic acid has a large molecular weight, there are problems that it has high stickiness when applied to the skin and poor feeling of use, and is difficult to penetrate into the skin. To solve these problems, many studies are being conducted to control the viscosity of the solution of high molecules.

However, these methods for controlling viscosity, like methods of lowering the viscosity of the polymer solution by causing a hydrolysis reaction of the polymer chain of hyaluronic acid through pH adjustment and catalyst addition, and thus reducing the molecular weight, had the limitation of not maintaining the characteristics of the high molecule of hyaluronic acid.

In order to solve the above-mentioned problems, the inventors of the present invention have confirmed that by applying high shear stress to a pair of polymer chains of highly viscous polymeric hyaluronic acid, they are transformed into single polymer chain (single helix) structures, and ionic aggregates are formed by intramolecular interactions between the single polymer chains.

Through this, it was confirmed that the volume of the polymeric hyaluronic acid chain is reduced and the volume of the polymeric hyaluronic acid in the solution is reduced, thereby significantly lowering the viscosity.

Hereinafter, the preparation method of the structure of polymeric hyaluronic acid according to an embodiment of the present invention will first be described, and then the structure of polymeric hyaluronic acid will be described in detail.

**Preparation method of the structure of polymeric hyaluronic acid**

As used herein, the term "polymeric hyaluronic acid structure" refers to a structure having a single polymer chain structure formed by applying shear stress to polymeric hyaluronic acid having a pair of polymer chain structures.

As used herein, the term "precursor" refers to a precursor or precursor used to produce the structure of polymeric hyaluronic acid according to the present invention. In other words, the precursor of the structure of polymeric hyaluronic acid according to the present invention refers to hyaluronic acid having a pair of polymeric chain structures to which no shear stress has been applied.

The structure of polymeric hyaluronic acid according to an embodiment of the present invention can be prepared by applying shear stress to a solution containing hyaluronic acid, which is a precursor of the structure.

The shear stress applied to the solution containing hyaluronic acid, which a precursor of the structure, may be either mechanical shear stress or ultrasonic wave application.

The mechanical shear stress may be applied by passing the solution through a column or filter paper filled with silica. Below, mechanical shear stress is explained in detail.

According to an embodiment of the present invention, the mechanical shear stress may be applied by passing a solution containing hyaluronic acid, which is a precursor of the structure, through a column filled with silica. When the solution containing hyaluronic acid passes through the column filled with silica, etc., hyaluronic acid, which is a precursor of the structure, undergoes very high shear stress as it passes through a physically narrow area.

The silica may be spherical or prismatic, but its shape is not limited.

The size of the silica may be 0.01 to 0.50 mm, 0.05 to 0.40 mm, or 0.06 to 0.30 mm. If the size of the silica is less than 0.01 mm or exceeds 0.50 mm, even if the solution containing hyaluronic acid passes through the column filled with silica, shear stress may not be applied and thus there may be no change in structure.

A negative pressure of 0.1 bar to 1.0 bar or 0.2 bar to 0.9 bar can be applied to the bottom of the column filled with silica. If the negative pressure applied to the bottom of the column filled with silica is less than 0.1 bar, as the time required for the solution containing hyaluronic acid to pass through the column increases, the preparation time of the polymeric hyaluronic acid structure according to the present invention may be delayed. In addition, if the negative pressure applied to the bottom of the column filled with silica exceeds 1.0 bar, the time required for the solution containing the hyaluronic acid to pass through the column is reduced, and thus the preparation time of the polymeric hyaluronic acid structure according to the present invention can be shortened, but an additional pumping equipment is required, and thus, preparation costs may be increased.

According to another embodiment of the present invention, the mechanical shear stress may be applied by passing the solution containing hyaluronic acid through one or more filter papers. When passing through the one or more filter papers, hyaluronic acid, which is a precursor of the structure, undergoes very high shear stress by passing through a physically narrow area.

The filter paper may be one filter paper or two or more filter papers. If the filter paper consists of two or more filter papers, the filter papers can be arranged by stacking them. If the filter paper consists of a plurality of two or more filter papers, a higher shear stress than that produced by a single filter paper can be provided.

The pore size of the filter paper may be 0.1 to 5.0 microns or 0.3 to 4.5 microns. If the pore size of the filter paper is less than 0.1 micron, the amount of the solution containing hyaluronic acid passed through or filtered through the filter paper is too small, and thus the production rate of the structure of polymeric hyaluronic acid according to the present invention may be reduced. If the pore size of the filter paper exceeds 5.0 microns, the solution containing the hyaluronic acid may simply pass through the filter paper and thus shear stress may not be effectively applied.

The shear stress may be applied using ultrasonic waves. Below, the application of ultrasonic waves will be described in detail.

According to an embodiment of the present invention, the shear stress may be applied by applying ultrasonic waves to a solution containing the hyaluronic acid.

If the ultrasonic waves are applied to the solution containing the hyaluronic acid, a pressure wave is generated, and shear stress may be applied to hyaluronic acid, which is a precursor of the structure, by the pressure wave.

The intensity of the applied ultrasonic waves may be 200 J/sec to 800 J/sec or 400 J/sec to 600 J/sec.

The energy applied per volume of the applied ultrasonic waves can be calculated as the intensity of the ultrasonic waves (J/sec) x the applied time (sec) / measured volume (ml).

According to an embodiment of the present invention, the energy applied per volume of ultrasonic waves applied to the solution containing the hyaluronic acid may be 100 J/ml to 90 kJ/ml. If the energy of the ultrasonic waves is less than 100 J/ml, since sufficient shear stress is not applied to the solution containing the hyaluronic acid, it may be difficult to form a structure. In addition, if the energy of the ultrasonic waves exceeds 90 kJ/ml, excessive heat may be applied to the solution containing the hyaluronic acid, thereby making it difficult to form a structure.

The ultrasonic waves may be applied at 10°C to 80°C for 10 seconds to 60 minutes. If the ultrasonic waves are applied at a temperature less than 10°C, there is no change in the solution containing the hyaluronic acid, and if the ultrasonic waves are applied at a temperature exceeding 80°C, a phase change may occur in the solution containing hyaluronic acid, thereby making it difficult to form a structure of polymeric hyaluronic acid according to the present invention. In addition, if the ultrasonic waves are applied for less than 10 seconds, there is no change in the solution containing hyaluronic acid, and if the ultrasonic waves are applied for a time exceeding 60 minutes, the structure of the solution containing the hyaluronic acid is deformed, thereby making it impossible to form the structure of polymeric hyaluronic acid according to the present invention.

According to another embodiment of the present invention, in the method of applying the shear stress, a column filled with silica can be combined with an ultrasonic wave generator. The column filled with silica may be placed inside an ultrasonic wave generator, or the column filled with silica and the ultrasonic wave generator may be separated and placed continuously.

For example, after pouring the solution containing the hyaluronic acid into the column filled with silica, ultrasonic waves can be applied by placing the column in an ultrasonic wave generator. Additionally, after ultrasonic waves are applied to the solution containing the hyaluronic acid, the solution may be passed through the column filled with silica.

## Structure of polymeric hyaluronic acid

The structure of polymeric hyaluronic acid according to the present invention can be prepared by the preparation method described above. Specifically, the structure of polymeric hyaluronic acid can take the form of a single polymer chain by applying shear stress to hyaluronic acid, which is a precursor of the structure.

The single polymer chain may have a structure containing the chemical structure of hyaluronic acid as shown in FIG. 2. The chemical structure of the hyaluronic acid may be a single polymer chain having one carboxyl anion (-COO-) group for each two cyclic hexoses.

The single polymer chain may form ionic aggregates due to intramolecular interactions and/or interactions between ionic clusters and the like as the distance between single chains becomes closer. The intramolecular interaction may be achieved through intramolecular hydrogen bonding, ionic bonding, etc. The interaction between the ion clusters and the like can be achieved through interaction between partial charges of carboxyl anions (-COO$^{\delta-}$) and sodium cations (Na$^{\delta+}$) in the sodium salt of hyaluronic acid.

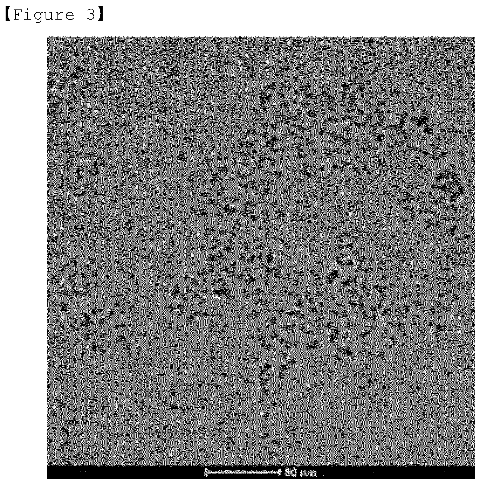

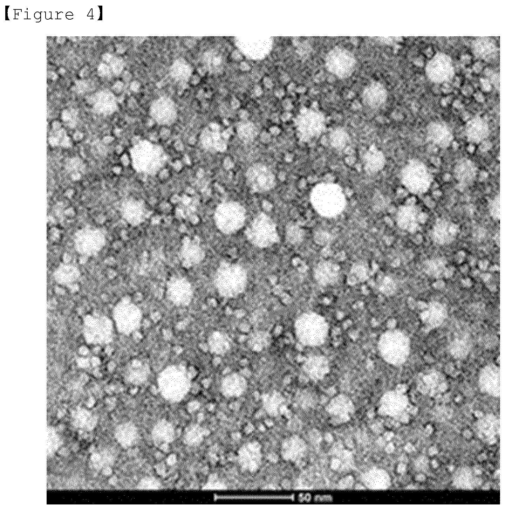

The ionic aggregate may include a spherical structure as shown in FIGS. 3 and 4. The ionic aggregate may include a spherical structure with a diameter of 1 to 20 nm, 2 to 18 nm, or 3 to 16 nm due to intramolecular interactions or interactions between ionic clusters and the like.

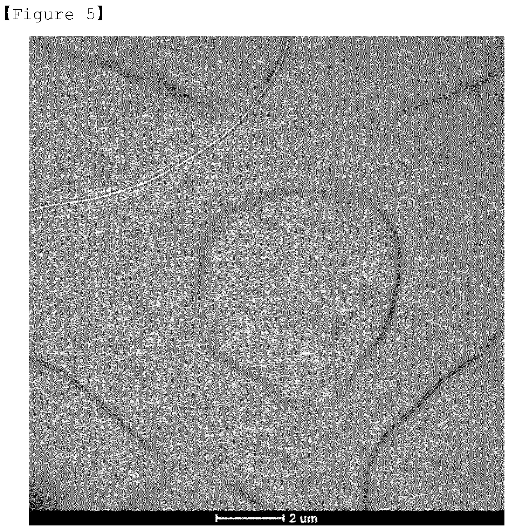

On the other hand, hyaluronic acid, which is a precursor of the structure according to the present invention, has a general polymer chain structure and does not contain ionic aggregates having the spherical structure. FIG. 5 is a TEM image of hyaluronic acid, which is a precursor of the structure according to the present invention, in which only lines due to marks generated during the freezing process for TEM imaging of the hyaluronic acid are observed, and the shape of the ionic aggregate having a spherical structure is not observed.

## Molecular weight of polymeric hyaluronic acid structure

The molecular weight of the polymeric hyaluronic acid structure according to the present invention may be $5.0 \times 10^4$ to $5.0 \times 10^6$ g/mol.

The structure of polymeric hyaluronic acid according to the present invention is the same as the molecular weight of hyaluronic acid which is a precursor of the structure. That is, even if a structure of polymeric hyaluronic acid is prepared by applying shear stress to a solution containing hyaluronic acid which is a precursor of the structure, it is possible to prepare a structure that maintains the same molecular weight as hyaluronic acid which is a precursor of the structure before applying shear stress, and has a lower viscosity.

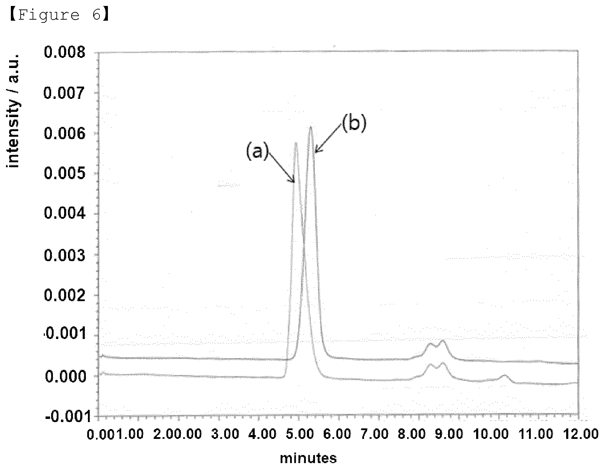

In FIG. 6, (a) shows the measurement curve of the molecular weight of the sodium salt of hyaluronic acid which is a precursor of the structure according to the present invention, and (b) shows the measurement curve of the molecular weight of the polymeric hyaluronic acid structure according to the present invention.

As shown in FIG. 6, it was confirmed that the measurement curve of the molecular weight of hyaluronic acid which is a precursor of the structure according to the present invention, and the measurement curve of the molecular weight of the polymeric hyaluronic acid structure according to the present invention are the same with almost no change. Therefore, the structure of polymeric hyaluronic acid according to the present invention does not reduce the molecular weight even when shear stress is applied to hyaluronic acid which is a precursor of the structure, and can provide a structure of polymeric hyaluronic acid with reduced viscosity.

The polymeric hyaluronic acid structure according to the present invention can have the effect of lowering the viscosity, relieving stickiness when applied to the skin, and improving the feeling of use, thereby improving penetration into the skin, while maintaining the same effects such as promoting collagen synthesis, excellent moisturizing power and the like, which are the characteristics of high molecular weight hyaluronic acid.

## Viscosity of polymeric hyaluronic acid structure

The viscosity of the solution containing the structure of polymeric hyaluronic acid, which has a molecular weight of $5.0 \times 10^4$ to $5.0 \times 10^6$ g/mol and a concentration of 0.1 to 2.0%, prepared according to an embodiment of the present invention may be 10 to 700 cP or 15 to 680 cP.

According to an embodiment of the present invention, if shear stress is applied to a solution containing polymeric hyaluronic acid which is a precursor of the structure of polymeric hyaluronic acid, the physical structure of hyaluronic acid is transformed from a twin helix structure into a single polymer chain, thereby lowering the viscosity while maintaining the high molecular weight of hyaluronic acid.

The reason why the viscosity of the solution containing the structure of polymeric hyaluronic acid according to the present invention is significantly lowered is that the single polymer chain is formed by applying shear stress to the solution containing hyaluronic acid which is a precursor of the structure, and the volume of the polymer chain can be reduced by forming ionic aggregates within the single polymer chains, and through this, the volume of the polymeric hyaluronic acid in the solution is reduced.

According to an embodiment of the present invention, when the molecular weight and concentration of the structure of the polymeric hyaluronic acid and hyaluronic acid which is a precursor of the structure are the same, a/b, which is the ratio of the viscosity (a) of the solution containing the structure and the viscosity (b) of the solution containing hyaluronic acid which is a precursor of the structure, may be 0.10 to 0.99.

### Contact angle of polymeric hyaluronic acid structure

The contact angle of the solution containing the structure of polymeric hyaluronic acid prepared according to an embodiment of the present invention may be 20° to 30° on the surface of hydrophilic glass and 80° to 100° on the surface of hydrophobic glass.

The measurement method of the contact angle may include a sessile drop method for measuring the static contact angle, and a tilting drop method, a captive drop method, and a wilhelmy plate method for measuring the dynamic contact angle, and preferably the sessile drop method may be used.

The surface of the hydrophilic glass may be a surface of glass containing a silica material that has not been treated at all.

The surface of the hydrophobic glass may be a surface of glass containing a material coated with Teflon emulsion.

### Intrinsic viscosity of polymeric hyaluronic acid structure

Intrinsic viscosity refers to the volume per unit mass of a high molecule when there is no viscous effect of the solution of high molecules.

In the method of measuring the intrinsic viscosity, intrinsic viscosity can be measured by calculating a reduced viscosity by measuring the aqueous solution to be measured according to its concentration, and then extrapolating this to read the reduced viscosity at concentration of 0. The intrinsic viscosity can be calculated using Equation 4 below.

Equation 2 below is an equation for calculating relative viscosity, and Equation 3 below is an equation for calculating reduced viscosity (wherein $\eta$ represents the viscosity of the solution, $\eta_0$ represents the viscosity of the solvent, and c represents the concentration of the solution). Generally, the concentration (c) of a solution uses the unit of (w/v) because the physical meaning of $\eta_{red}$ is the volume per unit weight of the high molecule in the solution. The reduced viscosity is graphed according to concentration, and the intercept obtained by linearly extrapolating the point where the concentration becomes 0, as described above, becomes the intrinsic viscosity.

[Equation 2]

$$\eta_{re} = \eta/\eta_0$$

(wherein, $\eta$ represents the viscosity of solution and $\eta_0$ represents the viscosity of solvent).

[Equation 3]

$$\eta_{red} = (\frac{\eta}{\eta_0} - 1)/c$$

(wherein, $\eta$ represents the viscosity of solution, $\eta_0$ represents the viscosity of solvent, and c represents the concentration of solution).

[Equation 4]

$$[\eta] = \lim_{c \to \infty} \frac{(\eta_{re} - 1)}{c}$$

(wherein, $\eta_{re}$ is the relative viscosity, and c is the concentration of solution).

The intrinsic viscosity of the solution containing the polymeric hyaluronic acid structure according to the present invention can be calculated based on Equations 2 to 4 above. The specific measured value of the intrinsic viscosity of solution containing the structure of polymeric hyaluronic acid according to the present invention is described in Experimental Example 9 below.

**Cosmetic composition containing polymeric hyaluronic acid structure**

According to an embodiment of the present invention, it is possible to provide a cosmetic composition containing the structure of the polymeric hyaluronic acid.

The cosmetic composition may contain the structural characteristics of polymeric hyaluronic acid according to the present invention.

The description of the structure of the polymeric hyaluronic acid is the same as described above.

Cosmetics prepared with the cosmetic composition of the present invention can be prepared in the form of general emulsified formulations and solubilized formulations. Cosmetics of emulsified formulation include nourishing toilet water, cream, and essence, and cosmetics of solubilized formulation include emollient toilet water. Also, in addition to the cosmetic composition of the present invention, it can be prepared in the form of an adjuvant for topical or systemic application commonly used in the cosmetic field by containing a dermatologically acceptable medium or base.

Suitable cosmetic formulations can be, for example, provided in the form of solutions, gels, solid or pasty anhydrous products, emulsions obtained by dispersing the oil phase in an aqueous phase, suspensions, microemulsions, microcapsules, creams, skins, lotions, powders, ointments, sprays or conceal sticks.

In addition, the cosmetic composition of the present invention may contain adjuvants commonly used in the field of cosmetics or dermatology, such as fatty substances, organic solvents, solubilizers, thickening and gelling agents, emollients, antioxidants, suspending agents, stabilizing agents, foaming agents, fragrances, surfactants, water, ionic or nonionic emulsifiers, fillers, metal ion sequestering and chelating agents, preservatives, vitamins, blocking agents, wetting agents, essential oils, dyes, pigments, hydrophilic or lipophilic actives or any other ingredients commonly used in cosmetics, in addition to the solution containing the structure of the polymeric hyaluronic acid. Additionally, the above ingredients can be introduced in amounts commonly used in the cosmetic field.

The cosmetic composition of the present invention can be prepared in any formulation commonly prepared in the art, and for example comprises cosmetics such as astringent toilet water, emollient toilet water, nourishing toilet water, various creams, essences, packs, foundations and the like, as well as cleansing products, facial cleansers, soaps, treatments, beauty essences, etc., and specific formulations of the cosmetic composition of the present invention comprise formulations such as skin lotion, skin softener, skin toner, astringent, lotion, milk lotion, moisture lotion, nourishing lotion, massage cream, nourishing cream, moisture cream, hand cream, essence, nourishing essence, pack, soap, shampoo, cleansing foam, cleansing lotion, cleansing cream, body lotion, body cleanser, emulsion, pressed powder, loose powder, and eye shadow.

Hereinafter, examples of the present invention will be described in detail so that those skilled in the art can easily practice the present invention. However, the present invention may be implemented in many different forms and is not limited to the examples described herein.

**Preparation of polymeric hyaluronic acid structure**

**[Example 1]**

Hyaluronic acid sodium salt (sodium hyaluronate, a product from Lyphar company, China) (0.5 g) with a molecular weight of $1.2 \times 10^6$ g/mol was placed in a mass cylinder, and distilled water was added to make the total volume of 100 ml. At 25°C, the solution was stirred until it became homogeneous to prepare an aqueous solution of hyaluronic acid sodium salt with a concentration of 0.5% (w/v).

A column with a diameter of 5.2 cm and a height of 6 cm was filled with 15 g of silica (Silica 60 from Merck company), and 100 g of distilled water was flowed to compact the silica in the column to prepare the column filled with silica. Afterwards, the prepared aqueous solution of hyaluronic acid sodium salt having a concentration of 0.5 (w/v)% was slowly flowed to the column filled with silica. A negative pressure of about 0.2 bar was applied to the bottom of the column filled with silica, and the aqueous solution of hyaluronic acid sodium salt was passed through the column (passage time: about 10 minutes) to prepare an aqueous solution containing the structure of polymeric hyaluronic acid.

**[Example 2]**

Hyaluronic acid sodium salt (sodium hyaluronate, a product from Lyphar company, China) (0.5 g) with a molecular weight of 1.2 x $10^6$ g/mol was placed in a mass cylinder, and distilled water was added to make the total volume of 100 ml. At 25°C, the solution was stirred until it became homogeneous to prepare an aqueous solution of hyaluronic acid sodium salt with a concentration of 0.5% (w/v).

After attaching a filter paper (Hyundai Micro company) with a pore size of 3 $\mu$m to the filter surface of the Buchner funnel, a negative pressure of about 0.2 bar was applied to the bottom of the Buchner funnel using a pressure reducing device, and the aqueous solution of hyaluronic acid sodium salt prepared above was filtered (filtration time: about 40 seconds) to prepare an aqueous solution containing the structure of polymeric hyaluronic acid.

**[Example 3]**

After stacking a filter paper with 3 gm pores (Hyundai Micro company), a filter paper with 1.0 gm pores, and a filter paper with 0.45 gm pores on the Buchner funnel, and then attaching it to the filter surface, a negative pressure of about 0.2 bar was applied to the bottom of the Buchner funnel using a pressure reducing device, and an aqueous solution containing the structure of polymeric hyaluronic acid was prepared in the same manner as in Example 2, except that the aqueous solution of hyaluronic acid sodium salt prepared as in Example 2 above was repeatedly filtered six times.

**[Example 4]**

Hyaluronic acid sodium salt (sodium hyaluronate, a product from Lyphar company, China) (1 g) with a molecular weight of 1.2 x $10^6$ g/mol was placed in a mass cylinder, and distilled water was added to make the total volume of 250 ml. At 25°C, the solution was stirred until it became homogeneous to prepare an aqueous solution of hyaluronic acid sodium salt with a concentration of 0.4% (w/v).

The aqueous solution of hyaluronic acid sodium salt prepared above was charged into an ultrasonic wave generator (JAC-5020 from Kodo Technical Research Company, Korea), and ultrasonic waves were applied at 25°C for 30 minutes to prepare an aqueous solution containing the structure of polymeric hyaluronic acid.

**[Example 5]**

An aqueous solution containing the structure of polymeric hyaluronic acid was prepared in the same manner as in Example 4 above, except that hyaluronic acid sodium salt with molecular weights of 3.0 x $10^4$ g/mol, 5.0 x $10^4$ g/mol, 1.0 x $10^5$ g/mol and 9.0 x $10^5$ g/mol is used.

**[Comparative Example 1]**

Hyaluronic acid sodium salt (sodium hyaluronate, a product from Lyphar company, China) (0.5 g) with a molecular weight of 1.2 x $10^6$ g/mol was placed in a mass cylinder, and distilled water was added to make the total volume of 100 ml. At 25°C, the solution was stirred until it became homogeneous to prepare an aqueous solution of hyaluronic acid sodium salt with a concentration of 0.5% (w/v).

**Experimental Example 1: Comparison of viscosity of Examples 1, 2 and Comparative Example 1**

The viscosity of the aqueous solutions of Examples 1 and 2 and Comparative Example 1 was measured at 25°C using a viscometer (DV2T viscometer from Brookfield company). The measured viscosity is listed in Table 1 below.

[Table 1]

|  | **Comparative Example 1** | **Example 1** | **Example 2** |
|---|---|---|---|
| **Viscosity (cP)** | 1,150 | 200 | 210 |

From the results in Table 1, it was confirmed that the viscosity of the aqueous solution containing the structure of polymeric hyaluronic acid prepared in Examples 1 and 2 is significantly lower than the viscosity of the solution of sodium salt of hyaluronic acid which is a precursor of the structure prepared in Comparative Example 1.

From this, it was confirmed that if a specific shear stress is applied to hyaluronic acid, the twin helix structure of hyaluronic acid is transformed into a single helix structure, and a structure of polymeric hyaluronic acid in which ionic aggregates are formed within the single polymer chain is prepared. The reason why the viscosity of the solution containing the structure of polymeric hyaluronic acid according to the present invention is significantly lowered is that by forming an ionic aggregate within the single polymer chain, the volume of the polymer chain can be reduced, thereby reducing the volume of the polymeric hyaluronic acid in the solution.

**Experimental Example 2: Measurement of viscosity according to concentration of aqueous solutions of Example 1 and Comparative Example 1**

Distilled water was added to the 0.5% (w/v) aqueous solution prepared in Example 1 and Comparative Example 1 to prepare aqueous solutions with various concentrations. The concentration of the aqueous solution was adjusted using distilled water only, without addition of other salts or adjustment of pH. The measured viscosity is listed in Table 2 below.

[Table 2]

| Concentration (%) | Comparative Example 1 | Example 1 |
|---|---|---|
| 0.1 | 37.97 cP | 16.95 cP |
| 0.2 | 140.0 cP | 38.01 cP |
| 0.3 | 370.9 cP | 90.0 cP |
| 0.4 | 645.3 cP | 127.20 cP |
| 0.5 | 1,150.0 cP | 195.0 cP |

From the results in Table 2, it was confirmed that in general, as the concentration is increased, the viscosity of the aqueous solution of polymeric hyaluronic acid is significantly increased (Comparative Example 1), while the aqueous solution containing the structure of polymeric hyaluronic acid according to the present invention maintains low viscosity even when the concentration is increased.

**Experimental Example 3: Measurement of viscosity according to molecular weight of aqueous solutions of Examples 4 and 5**

The viscosity of the structures prepared in Examples 4 and 5 was measured. This allowed comparison of the viscosity ratio before and after the application of ultrasonic waves according to molecular weight in the structure of hyaluronic acid with various molecular weights. At this time, the concentration of hyaluronic acid sodium salt prepared was 0.4% (w/v). The measured viscosity is listed in Table 3 below.

[Table 3]

| Molecular weight of hyaluronic acid sodium salt (g/mol) | Viscosity before application of ultrasonic waves (cp) | Viscosity after application of ultrasonic waves (cp) | Viscosity ratio before and after application of ultrasonic waves |
|---|---|---|---|
| $1.0 \times 10^4$ | 1.5 | 1.5 | 1.00 |
| $3.0 \times 10^4$ | 2.3 | 2.3 | 1.00 |
| $5.0 \times 10^4$ | 3.1 | 3.0 | 0.97 |
| $1.0 \times 10^5$ | 7.2 | 6.5 | 0.90 |
| $9.0 \times 10^5$ | 480 | 430 | 0.89 |
| $1.2 \times 10^6$ | 720 | 610 | 0.85 |
| $2.0 \times 10^6$ | 1200 | 910 | 0.76 |

From the results in Table 3, it was confirmed that the amount of change in viscosity before and after application of ultrasonic waves is increased as the molecular weight of hyaluronic acid sodium salt is increased. In addition, it was confirmed that in the structure of hyaluronic acid with a molecular weight ranging from $5.0 \times 10^4$ to $2.0 \times 10^6$ g/mol, the effect of reducing viscosity before and after application of ultrasonic waves is significant.

**Experimental Example 4: Measurement of viscosity of the aqueous solution in Example 4 according to the application time of ultrasonic waves**

The viscosity of the aqueous solution containing the structure of polymeric hyaluronic acid prepared in Example 4 was measured according to the application time of ultrasonic waves. The measured viscosity is listed in Table 4 below.

[Table 4]

| Application time (minutes) | Viscosity(cp) | Ratio before and after application |
|---|---|---|
| 0 | 720 | 1 (720/720) |
| 30 | 610 | 0.85 |
| 60 | 550 | 0.76 |
| 120 | 500 | 0.69 |

From the results in Table 4, it was confirmed that in hyaluronic acid sodium salt of the same molecular weight and concentration, the amount of change in viscosity before and after application of ultrasonic waves is increased as the time for applying ultrasonic waves is increased. That is, it was confirmed that in an aqueous solution containing the structure of hyaluronic acid with a molecular weight of $1.2 \times 10^6$ g/mol and a concentration of 0.4% (w/v), the longer the time for applying ultrasonic waves, the greater the effect of reducing viscosity before and after applying ultrasonic waves.

**Experimental Example 5: Measurement of change in molecular weight before and after application of ultrasonic waves**

An aqueous solution of hyaluronic acid sodium salt was prepared by adding hyaluronic acid sodium salt (0.02 g) with a molecular weight of $1.2 \times 10^6$ g/mol to a mass cylinder, and adding and dissolving 0.9% (w/v) NaCl so that the total volume was 20 mL. 0.8 mL of the aqueous solution was taken, 0.1 M $Na_2SO_4$ was added, and then the solution was prepared so that the total volume was 10 mL. Afterwards, distilled water was added to the solution to dilute the concentration two-fold to prepare a sample for High-Performance Liquid Chromatography (HPLC) analysis.

The solution prepared in the same manner as in Experimental Example 5 was charged into the ultrasonic wave generator, and ultrasonic waves were applied to prepare an aqueous solution containing the structure of polymeric hyaluronic acid. Using the aqueous solution prepared above, a sample for Gel Permeation Chromatography (GPC) measurement was prepared.

To analyze the concentration and molecular weight of the sample for HPLC analysis and GPC measurement, HPLC (e2695, waters) and GPC column (OHPak SB-802.5 HQ 8.0 x 300mm, 6um, Shodex) were used. A 0.1 M sodium sulfate ($Na_2SO_4$) aqueous solution prepared by dissolving 14.2 g of sodium sulfate anhydrous (DAEJUNG) in 1 L of purified water was used as the mobile phase. The column temperature was maintained at 40°C, the oven temperature was maintained at 25°C, 100% 0.1 M sodium sulfate ($Na_2S_{O4}$) was added, and the sample was stabilized in the oven for 1 hour at a flow rate of 1.0 mL/min. Afterwards, the sample was filtered with a syringe filter (material: PVDF, 0.2 um, $\Phi$ 13 mm), and then 20 $\mu$L of the obtained sample was injected into the GPC column and analyzed at a UV wavelength of 210 nm for 16 minutes.

In FIG. 6, (a) shows the molecular weight of hyaluronic acid sodium salt which is a precursor of the structure according to the present invention, and (b) shows the molecular weight of the structure of polymeric hyaluronic acid according to the present invention.

It was confirmed that the measurement curve of the molecular weight of hyaluronic acid which is a precursor of the structure according to the present invention, and the measurement curve of the molecular weight of the structure of polymeric hyaluronic acid according to the present invention are the same with almost no change.

**Experimental Example 6: Evaluation of skin spreadability and absorptivity of aqueous solutions of Examples 1, 2 and Comparative Example 1**

The aqueous solution containing the structure of hyaluronic acid according to the present invention can be applied in various fields. As an example, an aqueous solution containing the structure of hyaluronic acid can be used as a cosmetic composition.

Therefore, a sensory evaluation was conducted on the feeling of use when the aqueous solutions of Examples 1, 2, and Comparative Example 1 were applied to the skin, respectively. The skin spreadability and absorptivity of the aqueous solution according to the present invention were evaluated on 10 men and 10 women (total of 20 people).

The response to skin spreadability and absorptivity was defined in three levels as shown in Tables 5 and 6 below: skin spreadability (poor/ordinary/excellent) and absorptivity (poor/ordinary/excellent).

[Table 5]

| Response to spreadability | Comparative Example 1 | Example 1 | Example 2 |
|---|---|---|---|
| Poor | 10 | 0 | 0 |
| Ordinary | 9 | 2 | 3 |
| Excellent | 1 | 18 | 17 |

[Table 6]

| Response to absorptivity | Comparative Example 1 | Example 1 | Example 2 |
|---|---|---|---|
| Poor | 12 | 0 | 0 |
| Ordinary | 7 | 3 | 1 |
| Excellent | 1 | 17 | 19 |

From the results in Tables 5 and 6, it was confirmed that the aqueous solution containing the structure of hyaluronic acid according to the present invention exhibits excellent spreadability and absorptivity

**Experimental Example 7: Evaluation of moisturizing ability of the aqueous solutions of Examples 1, 2 and Comparative Example 1**

The water retention capacity of the aqueous solutions of Examples 1 and 2 and Comparative Example 1 was measured to evaluate the skin moisturizing ability.

1 g of each sample was collected, placed in a 10cc vial, and left at 25°C for 1 hour, and the weight change was measured. The results are shown in Table 7 below.

[Table 7]

| | Comparative Example 1 | Example 1 | Example 2 |
|---|---|---|---|
| **Weight reduction ratio** | 8.85% | 8.6% | 8.3% |

From Table 7, it was confirmed that although the viscosities of the aqueous solution of hyaluronic acid sodium salt (Example 1) passed through the column filled with silica and the aqueous solution of hyaluronic acid sodium salt (Example 2) passed through the filter were lower than the viscosity of the aqueous solution of hyaluronic acid sodium salt without shear stress (Comparative Example 1), the weight change was small. Through this, it was found that the aqueous solution containing the structure of hyaluronic acid according to the present invention had low viscosity and excellent moisturizing ability.

**Experimental Example 8: Measurement of contact angle of aqueous solutions of Examples 1 to 2 and Comparative Example 1**

The contact angle was measured for the aqueous solutions prepared in Examples 1 and 2 and Comparative Example 1. The contact angle was measured on the surface of hydrophilic glass and the surface of hydrophobic glass, respectively. The measured contact angles are listed in Table 8 below.

[Table 8]

| | Comparative Example 1 | Example 1 | Example 2 |
|---|---|---|---|
| **Contact angle on the surface of hydrophilic glass** | 19° | 20° | 23° |
| **Contact angle on the surface of hydrophobic glass** | 106° | 99° | 98° |

From Table 8, it was found that on the surface of hydrophilic glass, the contact angle of the aqueous solution prepared in Examples 1 and 2 is larger than that of Comparative Example 1, and on the surface of hydrophobic glass, the contact angle of the aqueous solution prepared in Example 1 and 2 is smaller than that of Comparative Example 1, so the aqueous solutions prepared in Examples 1 and 2 have relative lipophilicity.

**Experimental Example 9: Experiment on intrinsic viscosity**

The method of measuring intrinsic viscosity is performed by measuring the aqueous solution to be measured according to its concentration, calculating the reduced viscosity, and then extrapolating this to read the reduced viscosity at concentration of 0, and it is called intrinsic viscosity. As mentioned above, intrinsic viscosity refers to the volume per unit mass of high molecules when there is no interaction between polymer chains in the solvent.

The prepared 0.5 (w/v) aqueous solution of hyaluronic acid sodium salt was diluted with distilled water to concentrations of 0.3(w/v)%, 0.1(w/v)%, 0.05(w/v)%, 0.01 (w/v)% and 0.005 (w/v)%, respectively. The intrinsic viscosity is obtained by measuring the viscosity at 25°C using a Brookfield viscometer, DV2T viscometer, calculating it as reduced viscosity, and extrapolating. Hyaluronic acid sodium salt with a molecular weight of 1.2 million has an intrinsic viscosity of 2.3 $m^3$/kg, and hyaluronic acid sodium salt with a molecular weight of 900,000 has an intrinsic viscosity of 1.8 $m^3$/kg.

Hyaluronic acid sodium salt with a molecular weight of 1.2 million that passed through the silica-filled column prepared in Example 1 was calculated to have an intrinsic viscosity of approximately 1.15 $m^3$/kg and hyaluronic acid sodium salt with a molecular weight of 900,000 was calculated to have an intrinsic viscosity of approximately 0.8 $m^3$/kg.

**Claims**

1. A structure of polymeric hyaluronic acid, which is a structure derived from polymeric hyaluronic acid, and the structure has a single polymer chain.

2. The structure of polymeric hyaluronic acid according to claim 1, wherein the single polymer chain forms an ionic aggregate.

3. The structure of polymeric hyaluronic acid according to claim 2, wherein the ionic aggregate has a spherical structure with a diameter of 1 to 20 nm.

4. The structure of polymeric hyaluronic acid according to claim 1, wherein the molecular weight of the polymeric hyaluronic acid is in the range of 5.0 x $10^4$ to 5.0 x $10^6$ g/mol.

5. The structure of polymeric hyaluronic acid according to claim 1, wherein the structure is prepared by applying shear stress to a solution containing hyaluronic acid which is a precursor of the structure.

6. The structure of polymeric hyaluronic acid according to claim 5, wherein the shear stress is applied by passing a solution containing hyaluronic acid, which is a precursor of the structure, through a column filled with silica.

7. The structure of polymeric hyaluronic acid according to claim 5, wherein the shear stress is applied by passing a solution containing hyaluronic acid, which is a precursor of the structure, through one or more filter papers.

8. The structure of polymeric hyaluronic acid according to claim 7, wherein the filter paper is arranged by stacking two or more filter papers.

9. The structure of polymeric hyaluronic acid according to claim 5, wherein the shear stress is applied by applying ultrasonic waves to a solution containing hyaluronic acid which is a precursor of the structure.

10. The structure of polymeric hyaluronic acid according to claim 1, wherein when a solution containing the structure has a molecular weight of 5.0 x $10^4$ to 5.0 x $10^6$ g/mol and a concentration of 0.1 to 2.0%, the viscosity of the solution containing the structure is 10 to 700 cP.

11. The structure of polymeric hyaluronic acid according to claim 1, wherein when the structure and hyaluronic acid, which is a precursor of the structure, have the same molecular weight and concentration, a/b, which is the ratio of the viscosity (a) of the solution containing the structure and the viscosity (b) of the solution containing hyaluronic acid, which is a precursor of the structure, is 0.10 to 0.99.

12. The structure of polymeric hyaluronic acid according to claim 1, wherein the contact angle of the solution containing the structure is 20° to 30° on the surface of the hydrophilic glass.

13. The structure of polymeric hyaluronic acid according to claim 1, wherein the contact angle of the solution containing the structure is 80° to 100° on the surface of the hydrophobic glass.

14. The structure of polymeric hyaluronic acid according to claim 1, wherein the intrinsic viscosity of the solution containing the structure is 0.1 to 2.0 $m^3$/kg.

15. A cosmetic composition comprising the structure of polymeric hyaluronic acid according to claim 1.

16. The cosmetic composition according to claim 15, which is formed as any one formulation selected from the group consisting of skin lotion, skin softener, skin toner, astringent, lotion, milk lotion, moisture lotion, nourishing lotion, massage cream, nourishing cream, moisture cream, hand cream, essence, nourishing essence, pack, soap, shampoo, cleansing foam, cleansing lotion, cleansing cream, body lotion, body cleanser, emulsion, pressed powder, loose powder, and eye shadow.

17. A preparation method of a structure of polymeric hyaluronic acid, wherein the structure of the polymeric hyaluronic acid is prepared by applying shear stress to a solution containing hyaluronic acid which is a precursor of the structure.

18. The preparation method of a structure of polymeric hyaluronic acid according to claim 17, wherein the shear stress is applied by passing the solution containing hyaluronic acid, which is a precursor of the structure, through a column filled with silica.

19. The preparation method of a structure of polymeric hyaluronic acid according to claim 17, wherein the shear stress is applied by passing the solution containing hyaluronic acid, which is a precursor of the structure, through one or more filter papers.

20. The preparation method of a structure of polymeric hyaluronic acid according to claim 17, wherein the shear stress is applied by applying ultrasonic waves to hyaluronic acid which is a precursor of the structure.

【Figure 1】

【Figure 2】

【Figure 3】

【Figure 4】

【Figure 5】

【Figure 6】

## INTERNATIONAL SEARCH REPORT

| International application No. |
| --- |
| **PCT/KR2023/002352** |

| A. | CLASSIFICATION OF SUBJECT MATTER |
| --- | --- |

**C08B 37/08**(2006.01)i; **C08J 3/28**(2006.01)i; **A61K 8/73**(2006.01)i; **A61Q 19/00**(2006.01)i

According to International Patent Classification (IPC) or to both national classification and IPC

| B. | FIELDS SEARCHED |
| --- | --- |

Minimum documentation searched (classification system followed by classification symbols)

C08B 37/08(2006.01); A61K 45/00(2006.01); A61K 47/26(2006.01); A61K 47/36(2006.01); A61K 8/44(2006.01); A61K 8/73(2006.01); A61K 9/08(2006.01); A61Q 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히알루론산(hyaluronic acid), 저점도(low viscosity), 전단응력(shear stress), 단일 고분자 사슬(single polymer chain, single helix), 이온 회합체(ionic aggregate)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-2018-0014825 A (PRESBYOPIA THERAPIES, LLC) 09 February 2018 (2018-02-09)<br>See claim 9; and paragraphs [0072]-[0103]. | 1-3,5,11-14,17 |
| Y | | 4,6-10,15-16,18-20 |
| Y | KR 10-2279755 B1 (ULTRA V PHARM CO., LTD.) 20 July 2021 (2021-07-20)<br>See claims 1-19; and paragraphs [0003]-[0066]. | 4,10,15-16 |
| Y | JP 06-183998 A (MIWA, H. et al.) 05 July 1994 (1994-07-05)<br>See paragraph [0009]. | 6-9,18-20 |
| A | JP 2019-518789 A (BOSTON SCIENTIFIC SCIMED, INC.) 04 July 2019 (2019-07-04)<br>See entire document. | 1-20 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 May 2023** | **15 May 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/002352** |

**C.      DOCUMENTS CONSIDERED TO BE RELEVANT**

| Category* | Citation of document, with indication, where appropriate, of the relevant passages | Relevant to claim No. |
| --- | --- | --- |
| A | KR 10-2019-0002861 A (UCF-HYU (INDUSTRY-UNIVERSITY COOPERATION FOUNDATION HANYANG UNIVERSITY)) 09 January 2019 (2019-01-09)<br>See entire document. | 1-20 |

Form PCT/ISA/210 (second sheet) (July 2022)

## INTERNATIONAL SEARCH REPORT
### Information on patent family members

International application No.

**PCT/KR2023/002352**

| Patent document cited in search report | | | Publication date (day/month/year) | Patent family member(s) | | | Publication date (day/month/year) |
|---|---|---|---|---|---|---|---|
| KR | 10-2018-0014825 | A | 09 February 2018 | CN | 107847492 | A | 27 March 2018 |
| | | | | CN | 107847492 | B | 25 May 2021 |
| | | | | CN | 107920984 | A | 17 April 2018 |
| | | | | EP | 3310336 | A1 | 25 April 2018 |
| | | | | EP | 3310336 | A4 | 05 December 2018 |
| | | | | EP | 3310356 | A1 | 25 April 2018 |
| | | | | EP | 3310356 | A4 | 05 December 2018 |
| | | | | JP | 2018-517738 | A | 05 July 2018 |
| | | | | JP | 2018-517740 | A | 05 July 2018 |
| | | | | JP | 2021-035954 | A | 04 March 2021 |
| | | | | JP | 6835747 | B2 | 24 February 2021 |
| | | | | JP | 6838712 | B2 | 03 March 2021 |
| | | | | JP | 7026189 | B2 | 25 February 2022 |
| | | | | KR | 10-2018-0014824 | A | 09 February 2018 |
| | | | | US | 2015-0065511 | A1 | 05 March 2015 |
| | | | | US | 9089562 | B2 | 28 July 2015 |
| | | | | WO | 2016-205068 | A1 | 22 December 2016 |
| | | | | WO | 2016-205069 | A1 | 22 December 2016 |
| KR | 10-2279755 | B1 | 20 July 2021 | None | | | |
| JP | 06-183998 | A | 05 July 1994 | US | 6653293 | B1 | 25 November 2003 |
| JP | 2019-518789 | A | 04 July 2019 | CN | 109789219 | A | 21 May 2019 |
| | | | | CN | 113730605 | A | 03 December 2021 |
| | | | | EP | 3442589 | A1 | 20 February 2019 |
| | | | | JP | 2020-189215 | A | 26 November 2020 |
| | | | | JP | 2022-164780 | A | 27 October 2022 |
| | | | | JP | 6757464 | B2 | 16 September 2020 |
| | | | | JP | 7134202 | B2 | 09 September 2022 |
| | | | | US | 2018-0021252 | A1 | 25 January 2018 |
| | | | | WO | 2018-017861 | A1 | 25 January 2018 |
| KR | 10-2019-0002861 | A | 09 January 2019 | KR | 10-1969446 | B1 | 16 April 2019 |

Form PCT/ISA/210 (patent family annex) (July 2022)

**EP 4 484 452 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220023658

- KR 1020210015377